# EUROPÄISCHE PATENTANMELDUNG

(11) **EP 1 468 709 A1**
(43) Veröffentlichungstag der Anmeldung: **20.10.2004**
(21) Anmeldenummer: 04007793.5
(22) Anmeldetag: 31.03.2004
(51) Int. Cl.: A61N 5/10

(54) **Vorrichtung zur Simulation von Bestrahlungen für die Strahlentherapie**

(30) Priorität: 17.04.2003 DE 20306262 U
(71) Anmelder: Harmansa, Roman, 90592 Schwarzenbruck (DE)
(72) Erfinder: Harmansa, Roman, 90592 Schwarzenbruck (DE)
(74) Vertreter: Lösch, Christoph

(57) **Zusammenfassung**

Die Neuerung betrifft eine Vorrichtung zur Simulation von Bestrahlungen für die Strahlentherapie von Patienten mit einem aus wasseräquivalentem Material bestehenden Hohlkörper, der an mindestens einer Seite eine verschließbare Öffnung besitzt, wobei im Inneren des Hohlkörpers mindestens ein Erfassungselement 7 zur Ermittlung der Intensität, der Geometrie oder weiterer Parameter eines Strahlungsbildes aufnehmbar ist.

## Beschreibung

Die Neuerung betrifft eine Vorrichtung zur Simulation von Bestrahlungen für die Strahlentherapie von Patienten mit einem aus wasseräquivalentem Material bestehenden Hohlkörper.

Der Neuerung liegt die Aufgabe zugrunde, eine Vorrichtung zur Simulation von Bestrahlungen für die Strahlentherapie, insbesondere für die intensitäts-modulare Radio-Therapie (IMRT), anzubieten, welche einen verbesserten Bedienkomfort ermöglicht und flexibel gehandhabt werden kann.

Diese Aufgabe wird durch eine Vorrichtung mit den Merkmalen des Schutzanspruchs 1 gelöst. Vorteilhafte Ausführungsformen der Vorrichtung werden in den Unteransprüchen 2 - 18 beschrieben.

Die neuerungsgemäße Vorrichtung besteht aus einem Hohlkörper, der an mindestens einer Seite eine verschließbare Öffnung besitzt, wobei im Inneren des Hohlkörpers mindestens ein Erfassungselement zur Ermittlung der Intensität und der Geometrie eines Strahlungsbildes aufnehmbar ist.

Vor einer tatsächlichen Durchführung einer Strahlungstherapie wird die Strahlungsquelle auf eine derartige Vorrichtung zur Simulation von Bestrahlungen gerichtet und kann durch Auswertung der in der Vorrichtung aufgenommenen Erfassungselement festgestellt werden, welche räumliche Geometrie das Strahlungsfeld besitzt. Hierdurch kann sichergestellt werden, daß das Strahlungsfeld bei der tatsächlichen Durchführung der Strahlentherapie nur den Tumor erfaßt und umliegendes gesundes Gewerbe nicht von der Strahlung betroffen ist.

Vorteilhafterweise ist die Vorrichtung als Kubus ausgebildet und besitzt mehrere plattenartig ausgebildete Erfassungselemente, welche nach Art von Einschüben im Kubus aufgenommen sind. Die Erfassungselemente können dabei als Röntgenfilm ausgebildet sein, wobei insbesondere mehrere parallel zueinander angeordnete Röntgenfilme vorgesehen sind.

Nach einer weiteren vorteilhaften Ausführungsform können die Erfassungselemente auch zur Ionisationmessung sein.

Alternativ können die Erfassungselemente auch als Thermo-Lumineszenz-Dosimeter (TLD) ausgeführt sein, wobei derartige Platten mit einer Vielzahl von TLD-Sensoren versehen sein können.

Zur besseren Ausrichtung der Strahlungsquelle kann die neuerungsgemäße Vorrichtung äußere Markierungen aufweisen. Diese Markierungen können fest auf der Oberseite der Vorrichtung angebracht oder abnehmbar gestaltet sein.

Insbesondere bei der Verwendung von Röntgenfilmen als Erfassungselement besteht das Problem, daß die Röntgenfilme nach der Entwicklung wieder exakt in ihrer räumlichen Anordnung, in der sie in der Vorrichtung aufgenommen waren, ausgewertet werden müssen. Nur durch Einhaltung der räumlichen Anordnung kann die räumliche Struktur des Strahlungsbildes rekonstruiert und an die tatsächlichen Tumorabmessungen angepaßt werden.

Zur Markierung der einzelnen Röntgenfilme können die Distanzelemente, welche zur beabstandeten Anordnung der parallel zueinander angeordneten Röntgenfilme vorgesehen sind, optische Elemente aufweisen. Hierdurch kann Licht auf bestimmte Bereiche der einzelnen Röntgenfilme geleitet werden, wodurch die einzelnen Röntgenfilme an unterschiedlichen Stellen lokal belichtet werden, so daß abhängig von der derartig codierten Belichtung die Anordnung der einzelnen Röntgenfilme innerhalb des Kubus festgelegt wird.

Vorteilhafterweise besitzen die Distanzelemente zwischen den Röntgenfilmen Prismen zur Umleitung von Lichtstrahlen. Diese Prismen können sich über die gesamte Breite der Erfassungselemente erstrecken. In bestimmten Teilbereichen der Prismen werden nun Lichtstrahlen eingebracht, wobei für jeden Röntgenfilm in einem anderen Bereich ein Lichtstrahl angebracht wird. Damit wird jeder Röntgenfilm an einer einzigen bestimmten Stelle belichtet, so daß jeder Röntgenfilm eine individuelle Codierung aufweist, wodurch die einzelnen Röntgenfilme in ihrer jeweiligen Anordnung zueinander im nachhinein wieder rekonstruiert werden können.

Um Licht nur an bestimmten Stellen auf das optische Element einzuleiten, besitzt die neuerungsgemäße Vorrichtung vorteilhafterweise als Abdeckelement eine Blende mit einzelnen Bohrungen zum Lichtdurchtritt. Nach Aufstellen der Vorrichtung mit eingeschobenen Erfassungselementen mit optischen Elementen zur Umleitung von Licht und Abdeckung durch das geschilderte Blendenelement wird in einer Dunkelkammer ein einmaliger Belichtungsvorgang (Licht an/Licht aus) durchgeführt. Durch die Blenden gelangt Licht ins Innere der Vorrichtung, und zwar nur an den Stellen, an denen über die Blendenbohrungen ein Lichtdurchlaß gestattet wird. An diese Stellen wird das eingelassene Licht auch von den jeweiligen Prisen der Distanzelemente weitergeleitet, so daß ein Lichtpunkt auf den jeweils benachbart zu den Distanzelementen angeordneten Röntgenfilmen entsteht.

Nach Entfernen der Röntgenfilme aus der Vorrichtung ist dieser durch einen individuell gesetzten Lichtpunkt in seiner Lage zu benachbarten Röntgenfilmen identifizierbar.

Die Neuerung ist anhand von Ausführungsbeispielen in den Zeichnungsfiguren näher erläutert. Es zeigen:
- Fig. 1: eine Gesamtansicht einer als Kubus ausgebildeten Vorrichtung,
- Fig. 2: eine Begrenzungsfläche mit Markierung für einen Kubus nach Fig. 1,
- Fig. 3: eine Gesamtansicht einer als Kubus ausgebildeten Vorrichtung mit angebrachten Zusatzelementen,
- Fig. 4: ein Schnitt A-A durch Fig. 3,
- Fig. 5: ein Distanzelement mit einem optischen Element,
- Fig. 6: ein Schnitt B-B durch Fig. 5,
- Fig. 7: ein Detail X aus Fig. 6,
- Fig. 8: eine Abdeckung für einen Kubus,
- Fig. 9: ein Schnitt C-C durch Fig. 8,
- Fig. 10: ein Erfassungselement für eine Ionisationsmessung,
- Fig. 11: ein weiteres plattenförmiges Distanzelement,
- Fig. 12: eine Seitenansicht Y von Fig. 11 sowie
- Fig. 13: ein Erfassungselement mit TLD-Sensoren.

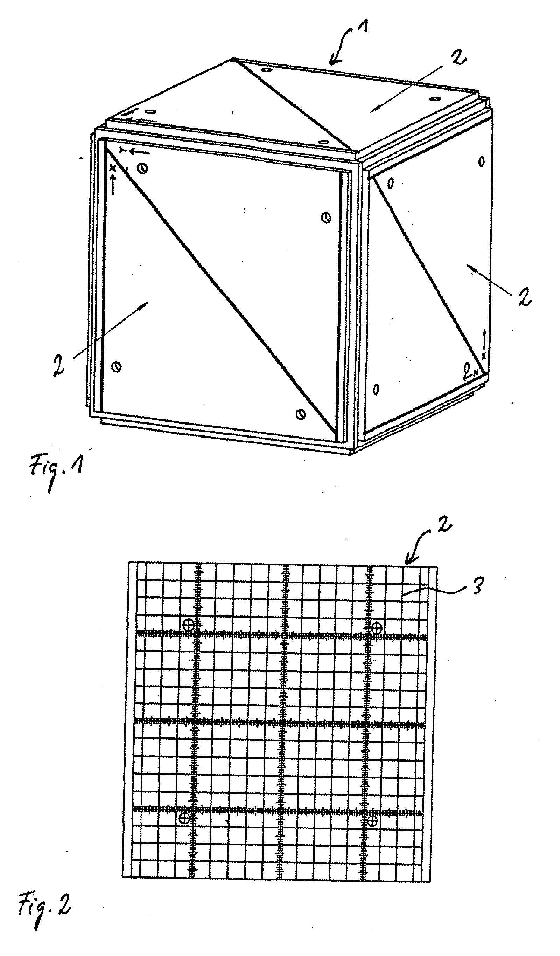

Fig. 1 zeigt einen Kubus 1 mit seitlichen Begrenzungsflächen 2. Auf den Begrenzungsflächen 2 sind Markierungen 3 (siehe Fig. 2) anbringbar, über welche eine Strahlungsquelle zur Bestrahlung des Kubus 1 ausgerichtet werden kann.

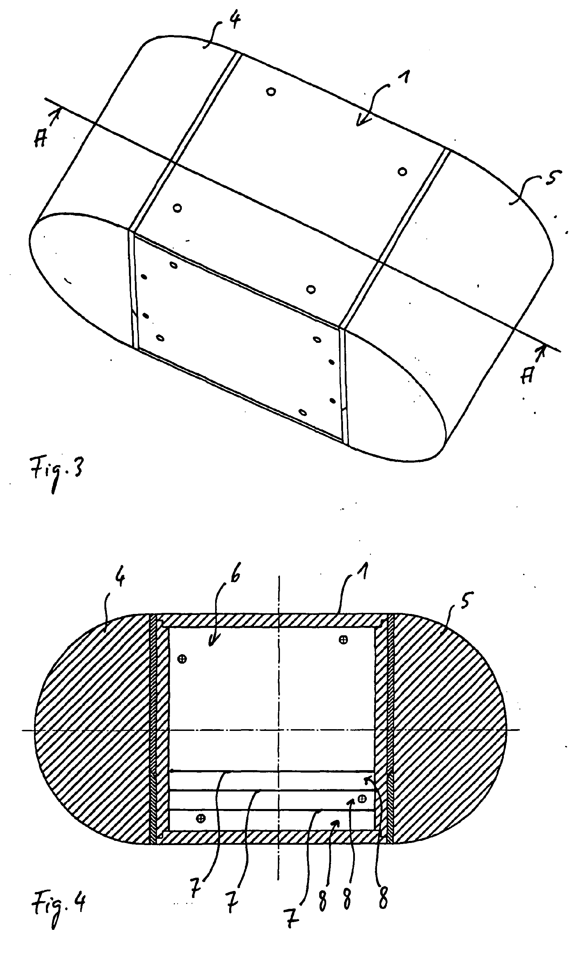

Der Kubus 1 nach Fig. 1 ist in Fig. 3 mit Zusatzelementen 4 und 5 versehen, um verschiedene geometrische Formen erzielen zu können, um damit die äußere Geometrie des zu bestrahlenden Organs oder Körperteils des Patienten weitestgehend simulieren zu können. Die Zusatzelemente 4 und 5 sind modular ausgebildet und können durch an sich bekannte Schnapp- oder Schraubverbindungen am Kubus 1 angebracht sein.

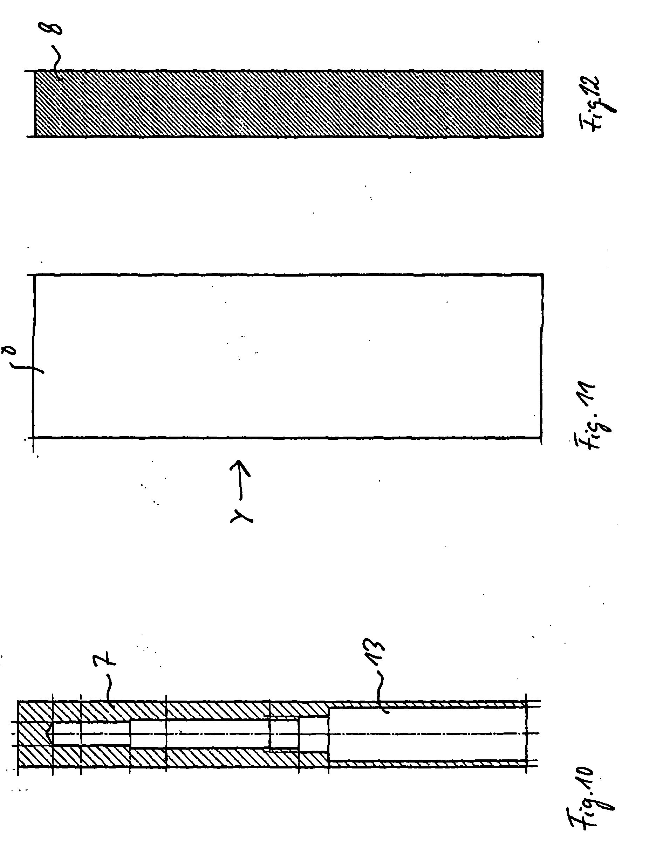

Im Schnitt A-A nach Fig. 4 wird der innere Hohlraum 6 des Kubus 1 gezeigt. In diesen Hohlraum 6 werden Erfassungselemente 7 (z.B. Röntgenfilme) eingelegt. Diese sind insbesondere über Distanzelemente 8 beabstandet zueinander angeordnet. Die Erfassungselemente 7 können mit Ausnehmungen 13 für einen Meßsensor zur Ionisationsmessung (siehe Fig. 10) ausgebildet sein oder Thermo-Lumineszenz-Dosimeter-Sensoren (TLD) 16 (siehe Fig. 13) aufweisen.

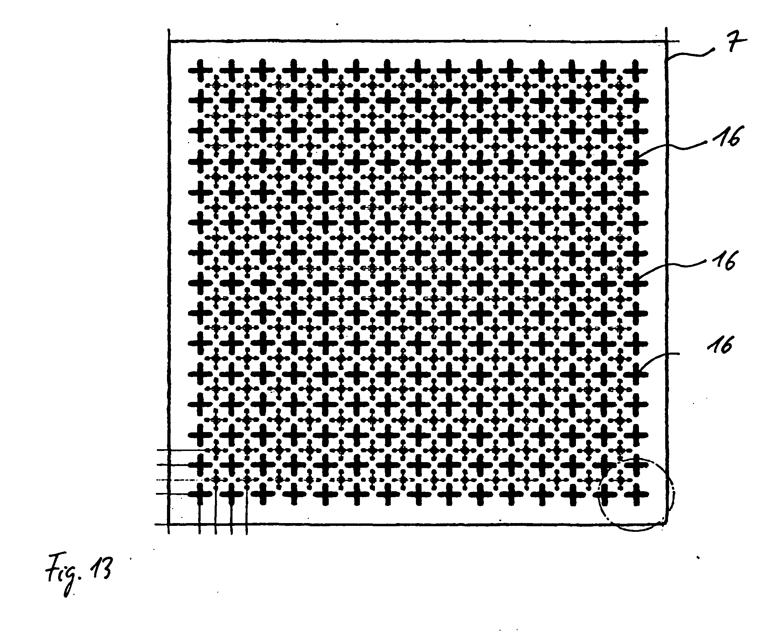

Generell kann der Hohlraum 6 des Kubus 1 mit insbesondere maßlich rasterförmig gestalteten und aufeinander abgestimmten plattenförmigen Erfassungselementen 7 und Distanzelementen 8 jeglicher Geometrie (vgl. z.B. Fig. 11 und 12) zum Einschieben vollständig ausgefüllt werden.

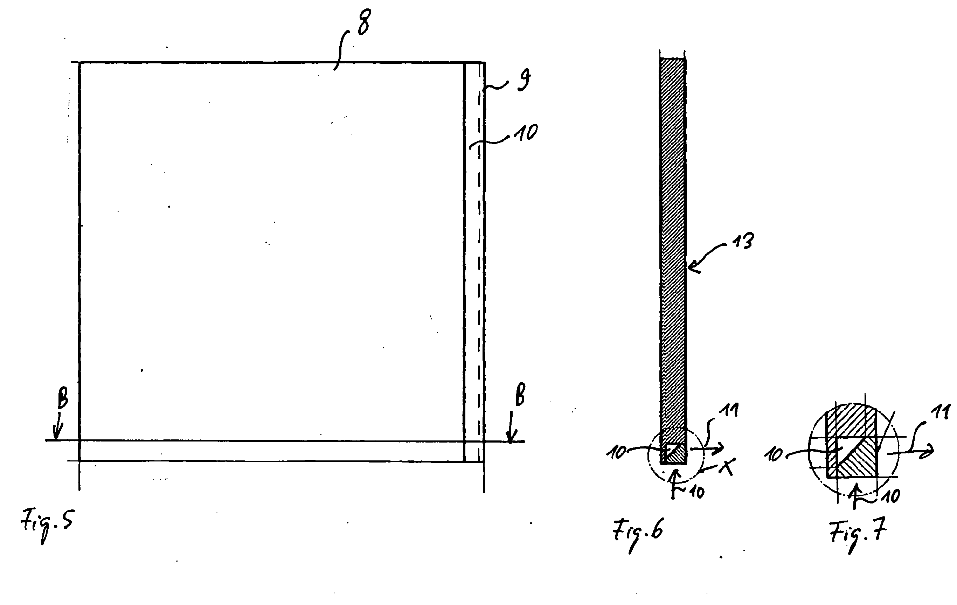

Ein weiteres Distanzelement 8 geht aus den Fig. 5 - 7 hervor. Dieses Distanzelement 8 besitzt vorteilhafterweise ein über die gesamte Längskante 9 sich ersteckendes optisches Element 10 zur Umlenkung eines Lichtstrahls. Das optische Element 10 kann z.B. als Prisma ausgebildet sein (vgl. Fig. 6). Dabei kann ein aus Richtung 11 eingeleiteter Lichtstrahl in Richtung 12 weitergeleitet werden. Somit kann ein auf der Oberfläche 13 angeordneter Röntgenfilm als Erfassungselement 7 in einem Randbereich belichtet werden.

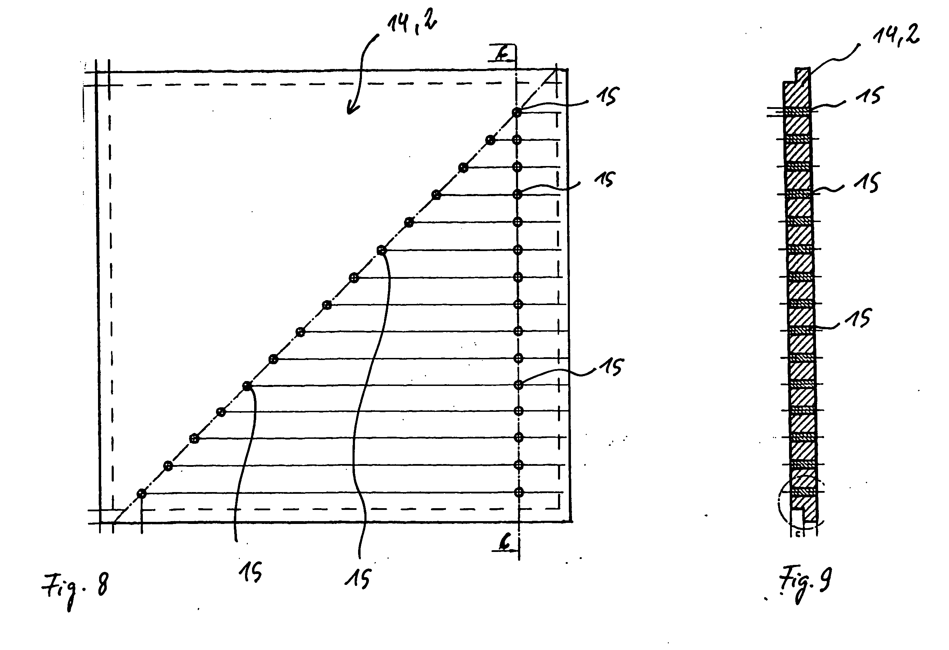

Die Fig. 8 und 9 zeigen eine Abdeckung 14 (Blende) des Kubus 1, welche eine der seitlichen Begrenzungsflächen 2 (vgl. Fig. 1) bilden. Diese Abdeckung 14 besitzt teilweise bezeichnete Bohrungen 15, durch die Licht ins Innere des Kubus 1 treten kann. Die Bohrungen 15 können als Öffnungen, Lichtleiter oder Dioden ausgebildet sein. Wenn nun durch die Bohrungen 15 Licht in den Kubus 1 eintritt und auf die optischen Elemente 10 der jeweiligen Distanzelemente 8 trifft, werden oberhalb der Oberflächen der Distanzelemente 8 angeordnete Röntgenfilme als Erfassungselemente 7 punktuell belichtet und erhalten somit eine Markierung. Damit kann nach der Belichtung der Röntgenfilme festgestellt werden, in welcher Reihenfolge sie zueinander angeordnet waren. Somit kann die räumliche Geometrie des Strahlungsbildes, welche die Belichtung auf den Röntgenfilmen erzeugt hat, rekonstruiert werden.

### BEZUGSZEICHEN

- 1: Kubus
- 2: Begrenzungsfläche
- 3: Markierung
- 4: Zusatzelement
- 5: Zusatzelement
- 6: Hohlraum
- 7: Erfassungselement
- 8: Distanzelement
- 9: Längskante
- 10: optisches Element
- 11: Richtung
- 12: Richtung
- 13: Ausnehmung
- 14: Abdeckung
- 15: Bohrung
- 16: TLD-Sensor

## Patentansprüche

1. Vorrichtung zur Simulation von Bestrahlungen für die Strahlentherapie von Patienten mit einem aus wasseräquivalentem Material bestehenden Hohlkörper, der an mindestens einer Seite eine verschließbare Öffnung besitzt, wobei im Inneren des Hohlkörpers mindestens ein Erfassungselement (7) zur Ermittlung der Intensität, der Geometrie oder weiterer Parameter eines Strahlungsbildes aufnehmbar ist.

2. Vorrichtung nach Anspruch 1, wobei die Vorrichtung die Form eines Kubus (1) besitzt.

3. Vorrichtung nach Anspruch 1 oder 2, wobei das Erfassungselement (7) plattenartig ausgebildet ist.

4. Vorrichtung nach Anspruch 3, wobei das Erfassungselement (7) als Röntgenfilm ausgebildet ist.

5. Vorrichtung nach Anspruch 4, wobei zwischen mehreren Röntgenfilm Distanzelemente (8) vorgesehen sind.

6. Vorrichtung nach Anspruch 5, wobei die Distanzelemente (8) plattenförmig ausgebildet sind.

7. Vorrichtung nach einem der vorhergehenden Ansprüche, wobei das Erfassungselement (7) für eine Ionisationsmessung ausgebildet ist.

8. Vorrichtung nach einem der vorhergehenden Ansprüche, wobei das Erfassungselement (7) als Thermo-Lumineszenz-Dosimeter-Sensor (TLD) (16) ausgebildet ist.

9. Vorrichtung nach einem der vorhergehenden Ansprüche, wobei an der Außenseite der Vorrichtung Markierungselemente (3) zur Ausrichtung des Strahlungsbildes angebracht sind.

10. Vorrichtung nach Anspruch 9, wobei die Markierungen (3) mit mindestens einer Begrenzungsfläche (2) der Vorrichtung verbindbar sind.

11. Vorrichtung nach einem der vorhergehenden Ansprüche, wobei an der Außenseite der Vorrichtung weitere Zusatzelemente (4,5) anbringbar sind.

12. Vorrichtung nach einem der vorhergehenden Ansprüche, wobei die Zusatzelemente (4,5) halb-zylinderförmig ausgebildet sind.

13. Vorrichtung nach einem der vorhergehenden Ansprüche, wobei die Distanzelemente (8) zwischen den Röntgenfilmen eine Optik zur Umlenkung eines Lichtstrahls aufweisen.

14. Vorrichtung nach Anspruch 13, wobei das optische Element (10) als Prisma ausgebildet ist.

15. Vorrichtung nach einem der Ansprüche 13 oder 14, wobei die Vorrichtung ein Deckelement mit Bohrungen (15) zum Lichtdurchtritt aufweist.

16. Vorrichtung nach Anspruch 15, wobei eine Lichtzuführung über die Bohrungen (15) zu den optischen Elementen (10) besteht.

17. Vorrichtung nach Anspruch 15 oder 16, wobei mehrere Bohrungen (15) parallel zu einer Kante (9) der Abdeckung (14) vorgesehen sind.

18. Vorrichtung nach einem der Ansprüche 15 - 17, wobei mehrere Bohrungen (15) diagonal von einem Eck der Abdeckung (14) zu einem gegenüberliegenden Eck der Abdeckung (14) verlaufend angeordnet sind.
